(19) Europäisches Patentamt
European Patent Office
Office européen des brevets

(11) **EP 2 367 493 B1**

(12) **EUROPÄISCHE PATENTSCHRIFT**

(45) Veröffentlichungstag und Bekanntmachung des Hinweises auf die Patenterteilung:
**27.08.2014 Patentblatt 2014/35**

(21) Anmeldenummer: **09743877.4**

(22) Anmeldetag: **28.10.2009**

(51) Int Cl.:
*A61B 18/12* (2006.01)      *A61B 18/16* (2006.01)
*A61B 5/01* (2006.01)       *A61B 5/053* (2006.01)

(86) Internationale Anmeldenummer:
**PCT/EP2009/007722**

(87) Internationale Veröffentlichungsnummer:
**WO 2010/049145 (06.05.2010 Gazette 2010/18)**

(54) **ELEKTROCHIRURGISCHES GERÄT MIT EINER TEMPERATURMESSEINRICHTUNG ZUR BESTIMMUNG EINER TEMPERATUR UND/ODER EINER TEMPERATURÄNDERUNG AN EINER NEUTRALELEKTRODE**

ELECTROSURGICAL DEVICE HAVING A TEMPERATURE MEASUREMENT DEVICE FOR DETERMINING A TEMPERATURE AND/OR A TEMPERATURE CHANGE AT A NEUTRAL ELECTRODE

APPAREIL ÉLECTROCHIRURGICAL DOTÉ D'UN DISPOSITIF DE MESURE DE TEMPÉRATURE POUR LA DÉTERMINATION DE LA TEMPÉRATURE ET/OU D'UNE MODIFICATION DE LA TEMPÉRATURE D'UNE ÉLECTRODE NEUTRE

(84) Benannte Vertragsstaaten:
**AT BE BG CH CY CZ DE DK EE ES FI FR GB GR HR HU IE IS IT LI LT LU LV MC MK MT NL NO PL PT RO SE SI SK SM TR**

(30) Priorität: **30.10.2008 DE 102008053841**
**19.03.2009 DE 102009013917**

(43) Veröffentlichungstag der Anmeldung:
**28.09.2011 Patentblatt 2011/39**

(73) Patentinhaber: **ERBE Elektromedizin GmbH**
**72072 Tübingen (DE)**

(72) Erfinder: **SELIG, Peter**
**72379 Hechingen (DE)**

(74) Vertreter: **Bohnenberger, Johannes**
**Meissner, Bolte & Partner GbR**
**Postfach 86 06 24**
**81633 München (DE)**

(56) Entgegenhaltungen:
EP-A1- 1 902 684      EP-A2- 1 707 151
DE-A1- 3 544 443      US-A- 4 416 277
US-A1- 2007 049 919

**Beschreibung**

Die Erfindung betrifft ein elektrochirurgisches Gerät gemäß Anspruch 1.

In der Hochfrequenzchirurgie wird elektrische Energie dem zu behandelnden Gewebe zugeführt. Hierbei wird generell zwischen einer monopolaren und einer bipolaren Anwendung des Hochfrequenzstroms (HF-Stroms) unterschieden.

Bei einer monopolaren Anwendung ist üblicherweise nur eine Aktivelektrode vorgesehen, an die eine hochfrequente Wechselspannung angelegt wird. Die Aktivelektrode befindet sich beispielsweise an einem elektrochirurgischen Instrument zum Schneiden und/oder Koagulieren von Gewebe. Ferner ist eine Anbringung einer Neutralelektrode am Körper des Patienten erforderlich, durch die der Stromkreis über das zwischen Aktivelektrode und Neutralelektrode liegende Gewebe geschlossen wird. Die Form der Aktivelektrode hängt von der jeweiligen Anwendung ab. Die Oberfläche der Aktivelektrode, über die Wechselstrom in das Gewebe geleitet wird, ist verhältnismäßig klein, so dass in der direkten Umgebung der Aktivelektrode eine hohe Stromdichte und demzufolge auch eine hohe Wärmeentwicklung entstehen.

Die Stromdichte nimmt mit dem Abstand von der Aktivelektrode stark ab, sofern nicht durch erhebliche Unterschiede in der Gewebeleitfähigkeit auch an weiteren Körperstellen hohe Stromdichten auftreten. Die an die Aktivelektrode angelegten Wechselspannung werden über die Neutralelektrode abgeleitet. Es ist darauf zu achten, dass die Neutralelektrode großflächig an dem Körper des Patienten anliegt und dem hochfrequenten Wechselstrom nur einen geringen Übergangswiderstand entgegensetzt.

Bei der bipolaren Anwendung sind zwei Aktivelektroden vorgesehen, zwischen denen das zu behandelnde Gewebe aufgenommen wird. Der Stromfluss wird über das zwischen den beiden Aktivelektroden liegende Gewebe geschlossen, so dass dieses bei einem Anlegen einer HF-Spannung erhitzt wird. Dabei fließt ein Großteil des Stroms zwischen den beiden Aktivelektroden.

Es kommt vor, dass die Neutralelektrode nicht korrekt am Patienten angebracht wird oder sich während der Behandlung teilweise ablöst. In diesen Fällen ist der Stromfluss auf die noch anliegenden Teile der Neutralelektrode beschränkt, was zu einer deutlich höheren Impedanz an dieser und allgemein zu einer höheren Stromdichte innerhalb des benachbarten Gewebes führen kann. Wie der nachfolgende Bezug auf Dokumente aus dem Stand der Technik zeigt, sind Überwachungssysteme bekannt, mit denen eine Beurteilung der Anbringungsqualität der Neutralelektrode möglich ist.

Beispielsweise ist aus der DE 10 2004 025 613 B4 ein Verfahren zur Bestimmung der Übergangsimpedanz zwischen zwei Teilelektroden oder Elektrodenabschnitten einer geteilten Neutralelektrode in der Hochfrequenzchirurgie bekannt. Hierbei werden mittels eines Schwingkreises die Übergangsimpedanzen zwischen den beiden Elektrodenabschnitten bestimmt. Es wird unterstellt, dass bei einem großflächigen Anliegen der Neutralelektrode die Übergangsimpedanz zwischen den einzelnen Abschnitten wesentlich geringer ist.

In letzter Zeit wurden Behandlungsmethoden entwickelt, bei denen relativ hohe HF-Ströme über eine längere Zeitdauer appliziert werden. Das Risiko einer Verbrennung des Gewebes an der Neutralelektrode ist bei diesem Verfahren erhöht. Somit kann es also auch bei einer korrekt angelegten Neutralelektrode je nach Behandlungsverfahren oder Behandlungsverlauf zu einer Schädigung des Gewebes kommen. Theoretisch wäre es zwar denkbar, die Anlagefläche der Neutralelektrode weiter zu vergrößern, jedoch ist dies oft nicht praktikabel.

Daher ist es notwendig, die Temperatur an der Neutralelektrode zu überwachen. Die US 2006/0079872 A1 stellt hierfür eine Vorrichtung bereit. Gemäß dieser Druckschrift wird ein Widerstand in den Behandlungsstrom eingekoppelt, dessen Erwärmung mittels eines Wärmesensors überwachbar ist. Der Widerstand soll so gewählt sein, dass er im Wesentlichen reale Impedanzverhältnisse zwischen der Neutralelektrode und der Aktivelektrode wiedergibt. Eine geeignete Wahl des Widerstands ist jedoch sehr schwierig, da sich die Impedanzwerte bei jeder Anwendung abhängig von der verwendeten Methode, dem Instrument, der Positionierung des Instruments und der Neutralelektrode, dem zu behandelnden Organ usw. ändern.

Es gibt weitere Ansätze, bei denen es angedacht ist, handelsübliche Temperatursensoren unmittelbar an den Elektroden vorzusehen. Das Bereitstellen dieser Messeinrichtungen an den Elektroden ist jedoch sehr aufwändig. Des Weiteren kommt es häufig zu lokalen Erwärmungen, die möglicherweise von diesen Sensoren nicht erfasst werden können.

In der Regel wird die Impedanz zwischen den beiden Hälften einer geteilten Neutralelektrode wie bereits beschrieben gemessen. Dies liefert einen Richtwert für die angelegte Fläche, da der Widerstand anti-proportional zu dieser ist. Des Weiteren wird der Strom über die Neutralelektrode gemessen und zusammen mit dem Übergangswiderstand wird eine theoretische Verlustleistung abgeschätzt, die an den Elektroden abfällt. Diese Verlustleistung lässt sich mit empirisch ermittelten Grenzwerten vergleichen, um Rückschlüsse auf die an der Neutralelektrode vorliegende Temperatur zu ziehen. Diese Ansätze sind jedoch sehr fehlerbehaftet und können keinen sicheren Schutz vor einer Schädigung des Patienten liefern. Unterschiedliche Gewebetypen werden hierbei nicht berücksichtigt.

Ausgehend von diesem Stand der Technik, insbesondere von der EP 1 707 151 A2, ist es Aufgabe der vorliegenden Erfindung, ein elektrochirurgisches Gerät mit einer verbesserten Temperaturmesseinrichtung anzugeben. Des Weiteren soll ein entsprechendes Verfahren zur Bestimmung einer Temperatur und/oder Temperaturänderung an einer Neutralelektrode bereitgestellt werden. Insbesondere sollen das Verfahren und die Vorrichtung eine sichere und effiziente Beurteilung der Temperaturverhältnisse an der Neutralelektrode ermöglichen.

Die Aufgabe wird erfindungsgemäß durch ein elektrochirurgisches Gerät gemäß dem Anspruch 1 gelöst.

Ein zentraler Gedanke besteht also darin, die Temperatur der Neutralelektrode oder deren Temperaturänderung anhand von Impedanzmessungen abzuschätzen. Hierfür weist die erfindungsgemäße Neutralelektrode eine Kontaktmittelschicht auf, die eine temperaturspezifische Impedanz hat. Der elektrische Widerstand der Kontaktmittelschicht ändert sich also in Abhängigkeit von der vorliegenden Temperatur. Unter einer temperaturspezifischen Impedanz im Sinne dieser Erfindung ist die Veränderung der Impedanz in Abhängigkeit von der Temperatur innerhalb eines relevanten Temperaturbereichs gemeint. Für die Elektrochirurgie liegt der relevante Temperaturbereich im Intervall zwischen 10 und 100°C. Möglicherweise reicht ein Intervall von 20 bis 70°C, insbesondere 20 bis 60°C aus.

Vorzugsweise wird die Kontaktmittelschicht eine derartige Materialeigenschaft aufweisen, dass die Impedanz mit zunehmender Temperatur abnimmt, insbesondere innerhalb des relevanten Intervalls. Bei lokalen Erwärmungen der Neutralelektrode kommt es zu einem Abfall der gemessenen Impedanz. Die Impedanzmesseinrichtung kann also stets die geringste Impedanz und somit den Abschnitt der Kontaktmittelschicht mit der höchsten Temperatur erfassen.

Die Impedanzmesseinrichtung umfaßt einen Messstromgenerator, der zur Bereitstellung eines Messstroms an einem ersten Elektrodenabschnitt und an einem zweiten Elektrodenabschnitt ausgebildet ist. Es wird also die Neutralelektrode in mindestens einen ersten Elektrodenabschnitt und einen zweiten Elektrodenabschnitt unterteilt. Die Messung der Impedanz wird zwischen diesen beiden Elektrodenabschnitten gewährleistet. Denkbar ist eine Messung einer Vielzahl von Impedanzen zwischen einer Vielzahl von Elektrodenabschnitten. Somit wird eine bessere Detailauflösung der Temperaturverhältnisse an der Neutralelektrode erzielt. Die Neutralelektrode dient also nicht nur zur Applikation des HF-Stroms, sondern auch zur Bestimmung der Impedanzen bzw. Impedanzverhältnisse innerhalb oder an der Kontaktmittelschicht.

Der Messstromgenerator kann zur Bereitstellung des Messstroms mit einer Wechselspannung, insbesondere mit einer Wechselspannung mit einer Frequenz $\leq 300$ kHz, insbesondere $\leq 150$ kHz, insbesondere $\leq 100$ kHz ausgebildet sein. Mit derart niedrigen Frequenzen im Vergleich zu denen, die bei der Behandlung mittels des HF-Stroms verwendet werden, kann eine effektive Messung der Impedanz erfolgen. Denkbar ist es, Messströme und HF-Ströme für die Behandlung durch entsprechende Filter voneinander zu trennen und getrennt auszuwerten.

Die Elektrodenabschnitte sind elektrisch voneinander isoliert auf der Kontaktmittelschicht angeordnet. Um unterschiedliche Potentiale an den einzelnen Elektrodenabschnitten zu generieren, ist es notwendig, diese derart auszubilden, dass sie voneinander elektrisch isoliert sind.

Der HF-Generator kann zur Bereitstellung eines HF-Stroms mit einer Wechselspannung mit einer Frequenz $\geq 300$ kHz, insbesondere $\geq 1000$ kHz ausgebildet sein. Derartige Frequenzen sind in der HF-Chirurgie üblich und geeignet, eine vorteilhafte Koagulation und Durchtrennung von Gewebe durchzuführen. Diese Frequenzen unterscheiden sich deutlich von den Frequenzen, die für die Messströme verwendet werden. Frequenzfilter können angewandt werden, um die Messspannung von der HF-Spannung zu trennen.

Die Kontaktmittelschicht kann eine elektrische Impedanz mit einer hohen Temperaturabhängigkeit, insbesondere mit einer (relativen) Impedanzänderung $\geq 1\%$ pro Grad Celsius, insbesondere $\geq 2\%$ pro Grad Celsius aufweisen. Je höher die Temperaturabhängigkeit der verwendeten Kontaktmittelschicht ist, umso einfacher lässt sich eine Temperaturänderung mittels der Impedanzänderung detektieren. Vorzugsweise ist die relative Impedanzänderung im relevanten Temperaturbereich größer als 1% pro Grad Celsius.

Die Kontaktmittelschicht kann Hydrogel umfassen oder aus diesem bestehen. Vorzugsweise ist die Kontaktmittelschicht aus Hydrogel ausgebildet. Bei der Applikation von HF-Strom wird Hydrogel verwendet, um den Übergangswiderstand zwischen den Elektroden und der Haut zu reduzieren. Hydrogel weist bezüglich seiner Impedanz eine starke Abhängigkeit von der Temperatur auf. Daher ist Hydrogel sehr gut geeignet, um die erfindungsgemäße Temperaturermittlung vorzunehmen. Das Hydrogel hat in diesem Fall eine Doppelfunktion. Zum einen dient es zur besseren Applikation des HF-Stroms und/oder mechanischen Befestigung der Neutralelektrode am Patienten, zum anderen bildet es einen Teil eines Temperatursensors.

Die Temperaturmesseinrichtung kann eine Impedanzintegrationseinrichtung umfassen, die dazu ausgebildet ist, Impedanzänderungen über einen vorgegebenen Zeitraum zu integrieren, um Wärmebilanzschätzungen vorzunehmen. Durch eine langfristige Beobachtung (zeitliche Integration) der Impedanzänderungen über alle Aufwärm- und Abkühlphasen im Verlauf eines Eingriffs, kann eine reale Wärmebilanzschätzung durchgeführt und somit eine zuverlässige Aussage über die thermische Situation an der Neutralelektrode gemacht werden.

Der vorgegebene Zeitraum kann eine Vielzahl von Aktivierungs- und Deaktivierungsphasen des HF-Generators umfassen. Somit kann sowohl die Erwärmung während der Aktivierungsphase - HF-Strom wird appliziert - wie auch die Abkühlung während der Deaktivierungsphase - kein HF-Strom wird appliziert - bei der Beurteilung der Temperatur berücksichtigt werden.

Das elektrochirurgische Gerät kann eine Erkennungsvorrichtung zur Bestimmung von Parametern, insbesondere mindestens einer Elektrodenfläche der Neutralelektrode und/oder eines Temperaturkoeffizienten umfassen. Die an einer Neutralelektrode gemessenen Impedanzwerte hängen von einer Vielzahl von Faktoren ab. Hierunter fallen die Fläche der Neutralelektrode, insbesondere die Fläche der Elektrodenabschnitte, deren Position zueinander, dem Gewebewiderstand usw. Es ist möglich, für die Berechnung der Temperatur relevante Parameter gerätespezifisch, insbesondere neutralelektrodenspezifisch, zu hinterlegen. Die Erkennungsvorrichtung kann diese Parameter bestimmen oder auslesen und in entsprechenden Modellen oder Berechnungen verarbeiten.

Die Erkennungsvorrichtung kann eine Datenbank mit einer Vielzahl von Parametern und einer Vielzahl von Neutralelektrodentypen umfassen, wobei die Erkennungsvorrichtung dazu ausgebildet ist, ein Anschließen eines bestimmten Neutralelektrodentypen zu erfassen und die Parameter demgemäß aus der Datenbank auszulesen. Die Bestimmung der angeschlossenen Neutralelektrode kann also automatisch erfolgen (z.B. über ein RFID-Tag, das sich an der Neutralelektrode befindet). Zahlreiche andere Verfahren zur Bestimmung des Neutralelektrodentypen sind denkbar. Möglich wäre es auch, den Neutralelektrodentypen vor der Behandlung manuell einzugeben oder eine Bestimmung der relevanten Parameter in einer vorgegebenen Teststellung vorzunehmen.

Das elektrochirurgische Gerät kann eine Unterbrechungseinrichtung umfassen, die dazu ausgebildet ist, beim Überschreiten eines vorgegebenen Impedanzänderung bzw. beim überschreiten einer vorgegebenen Temperatur an der Neutralelektrode den HF-Strom zu unterbrechen oder zu limitieren. Denkbar ist es auch, dass die Unterbrechungseinrichtung beim Unterschreiten eines vorgegebenen Impedanzwerts ein Warnsignal abgibt.

Das elektrochirurgische Gerät kann eine Kontaktmittelschicht mit derartiger Materialeigenschaft aufweisen, dass deren Impedanz mit zunehmender Temperatur abnimmt. Das heißt, es kann ein Material verwendet werden, das einen negativen Temperaturkoeffizienten hat. Somit ist es möglich, entlang der großflächigen Neutralelektrode Abschnitte mit besonders geringem Widerstand bedingt durch besonders hohe Temperaturen zu erfassen.

Die Temperaturmesseinrichtung zur Bestimmung der Temperatur und/oder der Temperaturänderung kann den Effektivwert des HF-Stroms, insbesondere des applizierten HF-Stroms, berücksichtigen. Beispielsweise ist es möglich, eine Relation zwischen Impedanzänderung und Effektivwert zu berechnen (z. B. $\Delta R/I_{HF}$), um eine Temperaturänderung und/oder einen Widerstand, insbesondere einen Gewebewiderstand oder einen Übergangswiderstand zwischen Elektrode und Gewebe, zu ermitteln. Der Widerstand kann unter anderem darüber Aufschluss geben, wie gut eine Neutralelektrode am Gewebe anliegt.

Das elektrochirurgische Gerät umfaßt eine Stromintegrationseinrichtung, die erfindungsgemäß dazu ausgebildet ist, einen Wert bezüglich des HF-Stroms, insbesondere den Effektivwert, über die Zeit, insbesondere einen vorgegebenen Zeitraum aufzusummieren, und die Summe zur Bestimmung der Temperatur und/oder der Temperaturänderung mit einer Impedanzänderung in Beziehung zu setzen. Es ist einfacher und mit geringern Fehlern behaftet, wenn sowohl die Impedanzänderung wie auch die Summe des applizierten HF-Stroms über ein vorgegebenes Zeitintervall betrachtet wird. Die einzelnen Werte können in Relation gesetzt werden (z. B. $\Delta R/\Sigma I_{HF}$), um Kenngrößen des Systems zu erfassen und Temperatur und/oder Temperaturänderungen festzustellen.

Ein Verfahren zur Bestimmung einer Temperatur und/oder einer Temperaturänderung an einer Neutralelektrode mit einer Kontaktmittelschicht wird offenbart wobei das Verfahren die folgenden Schritte umfasst:

a) Bestimmen mindestens eines Impedanzwerts der Kontaktmittelschicht;

b) Berechnen einer Temperaturänderung und/oder einer Temperatur an der Neutralelektrode mindestens anhand des Impedanzwerts.

Auch das Verfahren nutzt die Abhängigkeit der Impedanz der Kontaktmittelschicht von der vorliegenden Temperatur. Der unmittelbaren Nachbarschaft zwischen Kontaktmittelschicht und dem applizierenden Teil der Neutralelektrode wegen sowie der Nachbarschaft zwischen der Kontaktmittelschicht und dem Gewebe wegen findet ein schneller Wärmeaustausch statt. Es kann davon ausgegangen werden, dass die Gewebetemperatur des Gewebes, das unmittelbar unterhalb der Neutralelektrode liegt, im Wesentlichen gleich der Temperatur der Neutralelektrode und der Kontaktmittelschicht ist. Somit lässt sich eine realistische Abschätzung der Temperaturbilanz an der Neutralelektrode durchführen. Ein unzulässig starkes Erhöhen der Temperatur aufgrund des applizierten HF-Stroms kann erkannt und unterbunden werden.

Der Schritt a) kann mehrfach während einer Vielzahl von Aktivierungs- und Deaktivierungsphasen erfolgen, um eine Vielzahl von Impedanzwerten zu ermitteln. Somit kann der Temperaturverlauf bzw. die einzelnen Temperaturveränderungen an der Neutralelektrode besser beurteilt werden. Es kann eine realistische Abschätzung der vorliegenden Temperatur durchgeführt werden.

Bei dem Schritt b) kann die Dauer der Aktivierungs- und/oder Deaktivierungsphase und/oder ein Effektivwert des HF-Stroms berücksichtigt werden.

Der Schritt b) kann eine Integration über eine Vielzahl von Impedanzwerten über die Zeit umfassen.

Das Verfahren kann ein Erfassen einer Impedanzänderung während einer Aktivierungs- und/oder Deaktivierungsphase umfassen. Beispielsweise ist es möglich, anhand des Quotienten zwischen Abkühlungszeit und Impedanzänderung Rückschlüsse auf die vorliegende Temperatur zu ziehen. Es kann wohl davon ausgegangen werden, dass die Neutralelektrode bei einem starken Temperaturgefälle zwischen Neutralelektrode und Umgebung schneller abkühlt. Die Impedanzänderung in Relation zur Zeit kann somit einen wichtigen Parameter zur Bestimmung der Temperatur bereitstellen.

Die Berechnung der Temperaturänderung kann eine lineare Abschätzung umfassen, insbesondere mittels der Formel:

$$\Delta T = \frac{R(T) - R(T_0)}{\alpha * R(T_0)} \quad ,$$

wobei

$\alpha$ :ein spezifischer Temperaturkoeffizient,
$T_0$:eine Ausgangstemperatur,
$R(T_0)$:eine Impedanz bei der Ausgangstemperatur $T_0$,
$R(T)$:die gemessene Impedanz ist.

Obwohl eine lineare Relation zwischen Temperatur und Impedanz bei dem verwendeten Kontaktmittel, vorzugsweise Hydrogel, nicht vorliegt, kann die Veränderung der Impedanz in Abhängigkeit zur Temperatur durch eine lineare Gleichung ausreichend genau angenähert werden. Alternativ können Polynomgleichungen mit einem höheren Grad zur Annäherung verwendet werden. Der spezifische Temperaturkoeffizient kann vorab in entsprechenden Teststellungen ermittelt werden. Denkbar ist es auch, eine Vielzahl von Temperaturkoeffizienten für eine Polynomgleichung mit einem höheren Grad zu ermitteln.

Das Verfahren kann umfassen:

- Ein Detektieren eines bestimmten Neutralelektrodentypen der angeschlossenen Neutralelektrode;
- Eine Auswahl eines vorgegebenen Temperaturkoeffizienten oder eines beliebigen anderen Parameters gemäß dem Neutralelektrodentypen.

Somit können vorab ermittelte Parameter automatisch in das Verfahren eingebunden werden.

Das Verfahren kann ein Ausgeben eines Warnsignals und/oder ein Abschalten oder Herunterregeln des HF-Stroms umfassen, wenn die gemessene Impedanzänderung einen vorgegebenen Grenzwert überschreitet. Somit kann bei einer unzulässigen Temperatur eine Warnmeldung ausgegeben, der HF-Strom unterbrochen oder limitiert werden, um eine Schädigung des Patienten auszuschließen.

Nachfolgend wird die Erfindung anhand von einigen Ausführungsbeispielen beschrieben, die mittels Abbildungen näher erläutert werden. Hierbei zeigen:

- Fig. 1 ein HF-Generatorsystem mit einem monopolaren Instrument;

- Fig. 2 Komponenten des HF-Generatorsystems;

- Fig. 3 Widerstands- und Stromverhältnisse an einer Neutralelektrode; und

- Fig. 4 ein Diagramm mit idealisierten Widerstands-Temperaturverläufen.

In der nachfolgenden Beschreibung werden für gleiche und gleich wirkende Teile dieselben Bezugsziffern verwendet.

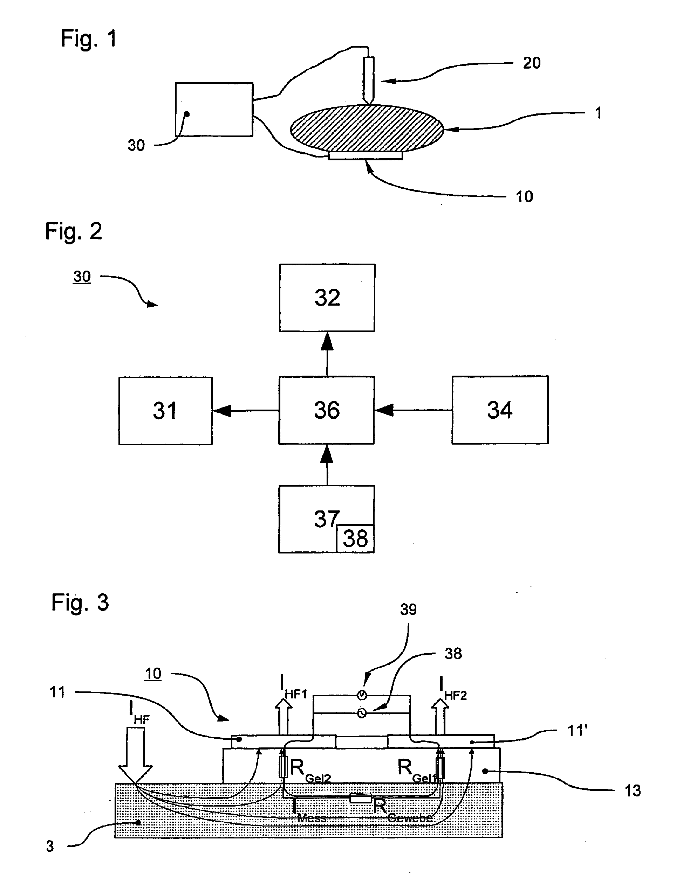

Die Fig. 1 zeigt ein elektrochirurgisches Gerät, umfassend ein HF-Generatorsystem 30, ein monopolares Instrument 20 und eine Neutralelektrode 10. Das HF-Generatorsystem 30 stellt einen HF-Strom $I_{HF}$ bereit, der mittels des monopolaren Instruments 20 und der Neutralelektrode 10 an einen Torso 1 appliziert wird. Die Fig. 1 stellt einen schematischen Querschnitt durch diesen Torso 1 dar. Die Neutralelektrode 10 ist großflächig an den Torso 1 angebracht. Das monopolare Instrument 20 beherbergt eine Aktivelektrode, die eine wesentlich geringere Fläche als die Neutralelektrode 10 aufweist. Der Strom fließt von der Aktivelektrode zur Neutralelektrode. In unmittelbarer Nähe der Aktivelektrode ist die Stromdichte so hoch, dass eine gezielte Koagulation oder Durchtrennung von Gewebe 3 (vgl. Fig. 3) durchgeführt werden kann.

Fig. 2 zeigt wesentliche Komponenten des HF-Generatorsystems 30. Diese umfassen eine Steuereinrichtung 36, eine Anzeigeeinrichtung 32, eine Bedieneinrichtung 34 und eine Messeinrichtung 37. Der Benutzer des elektrochirurgischen Geräts kann über die Bedieneinrichtung 34 den HF-Strom $I_{HF}$ aktivieren oder deaktivieren. Des Weiteren ist es möglich, verschiedene Betriebsmodi, beispielsweise einen zum Schneiden von Gewebe und einen anderen zum Koagulieren desselben einzustellen. Je nach den Eingaben des Benutzers, steuert die Steuereinrichtung 36 den HF-Generator 31, der einen HF-Strom $I_{HF}$ den Vorgaben entsprechend bereitstellt. Die Anzeigeeinrichtung 32 kann dazu verwendet werden, eingestellte Parameter, beispielsweise den aktuellen Betriebsmodus anzuzeigen. Des Weiteren kann die Anzeigeeinrichtung 32 eine aktuell an der Neutralelektrode 10 vorliegende Temperatur anzeigen und Warnhinweise ausgeben, die den Patienten vor einer ungewollten Schädigung durch die Behandlung schützen. Erfindungsgemäß wird die Temperatur der Neutralelektrode 10 durch die Messeinrichtung 37 unter Verwendung einer Sekundärstromquelle 38 ermittelt. Sobald die Neutralelektrode 10 eine Temperatur hat, die möglicherweise zu Verbrennungen führen könnte, wird der HF-Generator 31 abgeschaltet und die Anzeigeeinrichtung 32 gibt entsprechende Warnhinweise aus.

In einem Ausführungsbeispiel der erfindungsgemäßen Neutralelektrode 10 (vgl. Fig. 3) umfasst diese einen ersten Elektrodenabschnitt 11 und einen zweiten Elektrodenabschnitt 11'. Die Elektrodenabschnitte 11, 11' sind auf einem Trägermaterial derart angeordnet, dass diese gegeneinander elektrisch isoliert sind.

In einer Ausgestaltung der Neutralelektrode 10 befindet sich zwischen den einzelnen Elektrodenabschnitten 11, 11' ein elektrischer Isolator oder Hydrogel 13. Im vorliegenden Ausführungsbeispiel handelt es sich um eine selbstklebende Neutralelektrode 10, die eine Schicht aus elektrisch leitfähigem Hydrogel 13 aufweist und zur Applikation des HF-Stroms $I_{HF}$ auf ein Gewebe 3 aufgeklebt ist. Die vorliegende Erfindung macht es sich zu Nutzen, dass das Hydrogel 13 einen starken Temperaturkoeffizienten der Impedanz hat. Beispielsweise wurde bei handelsüblichen Neutralelektroden 10 mit handelsüblichem Hydrogel 13 im Temperaturbereich von 25°C bis 40°C eine relative Impedanzänderung von 2 bis 4% pro Grad Celsius gemessen. Dieser Effekt kann zur Bestimmung der Temperaturerhöhung an der Neutralelektrode 10 genutzt werden. Hierbei müssen allerdings verschiedene weitere Parameter berücksichtigt werden. Beispielsweise haben die Umgebungsbedingungen einen starken Einfluss auf die gemessene Impedanz R(T).

Zur Messung der von der Temperatur T abhängigen Impedanz R (T) umfasst die Messeinrichtung 37 die Sekundärstromquelle 38. Diese stellt einen Messstrom $I_{Mess}$ bereit, der an den Elektrodenabschnitten 11, 11' angelegt wird. Mittels einer parallel zur Sekundärstromquelle 38 geschalteten Spannungsmesseinrichtung 39 lässt sich eine Messspannung $U_{Mess}$ ermitteln. Somit kann die Messeinrichtung 37 eine Gesamtimpedanz messen. In einem ersten Modell wird davon ausgegangen, dass sich diese Gesamtimpedanz, wie in der Fig. 3 gezeigt, aus mehreren Widerständen zusammensetzt. So durchläuft der Messstrom $I_{Mess}$ von dem ersten Elektrodenabschnitt 11 das Hydrogel 13, tritt zumindest teilweise in das Gewebe 3 ein, durchläuft erneut das Hydrogel 13 und erreicht den zweiten Elektrodenabschnitt 11'. Die Gesamtimpedanz setzt sich also zumindest aus einem Gelwiderstand $R_{Gel1}$, einem Gewebewiderstand $R_{Gewebe}$ und einem zweiten Gelwiderstand $R_{Gel2}$ zusammen.

In dem ersten Modell kann davon ausgegangen werden, dass die Gewebewiderstandänderung im relevanten Temperaturenbereich (ca. 20 bis 70°) vernachlässigt werden kann. Die Messeinrichtung 37 kann die Gelwiderstände $R_{Gel1}$, $R_{Gel2}$ mittels des Messstroms $I_{Mess}$ ermitteln. Der Gewebewiderstand $R_{Gewebe}$ kann durch weitere Messungen ermittelt werden oder auf einen konstanten Wert gesetzt werden, der in Gewebe üblicherweise auftretenden ungefähren Widerständen entspricht.

In einem zweiten Modell geht man davon aus, dass die Gelwiderstände $R_{Gel1}$, $R_{Gel2}$ geringer sind als der Gewebewiderstand $R_{Gewebe}$, so dass die Messung der Messeinrichtung 37 ausschließlich die Veränderungen der Impedanz R(T) des Hydrogels 13 erfasst. Es ist möglich ein Hydrogel 13 entsprechend auszuwählen.

In einem dritten Modell, das wohl die Realität bei der Verwendung eines üblichen Hydrogels 13 am besten modelliert, unterstellt man, dass der Widerstand des Hydrogels 13 größer ist als der des Gewebes 3. Insbesondere wegen der geringen Schichtdicke des Hydrogels 13 kann dies in der Realität häufig auftreten. Experimente haben ergeben, dass 30% des Stromflusses innerhalb der Hydrogelschicht stattfindet während 70% des Stromflusses im Gewebe stattfindet. Es sind Situationen denkbar, bei denen nur ca. 10% des Stromflusses im Hydrogel 13 stattfindet. Die Impedanz R (T) setzt sich also, wie in Fig. 3 gezeigt, aus den Gelwiderständen $R_{Gel1}$, $R_{Gel2}$ und dem Gewebewiderstand $R_{Gewebe}$ zusammen. Da sich die Gewebetemperatur bei der Applikation des HF-Stroms $I_{HF}$ im Vergleich zu der Temperatur des Hydrogels 13 nur sehr langsam verändert - die Blutzirkulation führt zu einem schnellen Abtransport der anfallenden Wärmeenergie - kann auch bei diesem Modell ein konstanter oder annähernd konstanter Wert für $R_{Gewebe}$ unterstellt werden. Auf die Impedanzänderung $\Delta_R$ hat die Temperatur des Gewebes 3 nur einen geringen Einfluss. Diese lässt sich also erfindungsgemäß erfassen.

Da die Gelwiderstände $R_{Gel1}$, $R_{Gel2}$ mit zunehmender Temperatur T stark abnehmen, stellt sich ein weiterer vorteilhafter Effekt ein. Bei einer punktuellen oder lokalen Erwärmung der Neutralelektrode 10 kann ein starker Abfall der gemessenen Impedanz R(T) in diesem Bereich detektiert werden.

Der thermische Effekt, der sowohl im Gewebe 3 als auch im Hydrogel 13 und an der Neutralelektrode 10 auftritt, ist auf den angelegten HF-Strom $I_{HF}$ zurückzuführen. Bei der Verwendung von zwei Elektrodenabschnitten 11, 11' teilt sich dieser HF-Strom $I_{HF}$ in zwei HF-Teilströme $I_{HF1}$, $I_{HF2}$ auf. Diese HF-Teilströme $I_{HF1}$, $I_{HF2}$ sind schematisch in der Fig. 3 dargestellt.

In diesem Ausführungsbeispiel wird davon ausgegangen, dass sich die Beziehung zwischen der Impedanz $R(T)$ des Hydrogels 13 und dessen Temperatur T mit einem Temperaturkoeffizienten erster Ordnung $\alpha$ ausreichend genau modellieren lässt. Alternativ können Temperaturkoeffizienten höherer Ordnung hinzugenommen werden.

Mathematisch betrachtet stellt sich die Temperaturänderung $\Delta T$ wie folgt dar:

$$\Delta T = \frac{R(T) - R(T_0)}{\alpha * R(T_0)}$$

Hierbei ist $R(T)$ die gemessene Impedanz bei der Temperatur T, $R(T_0)$ eine Impedanz bei einer Ausgangstemperatur $T_0$ und $\alpha$ der spezifische Temperaturkoeffizient. Der spezifische Temperaturkoeffizient $\alpha$ lässt sich beispielsweise innerhalb eines Testaufbaus ermitteln.

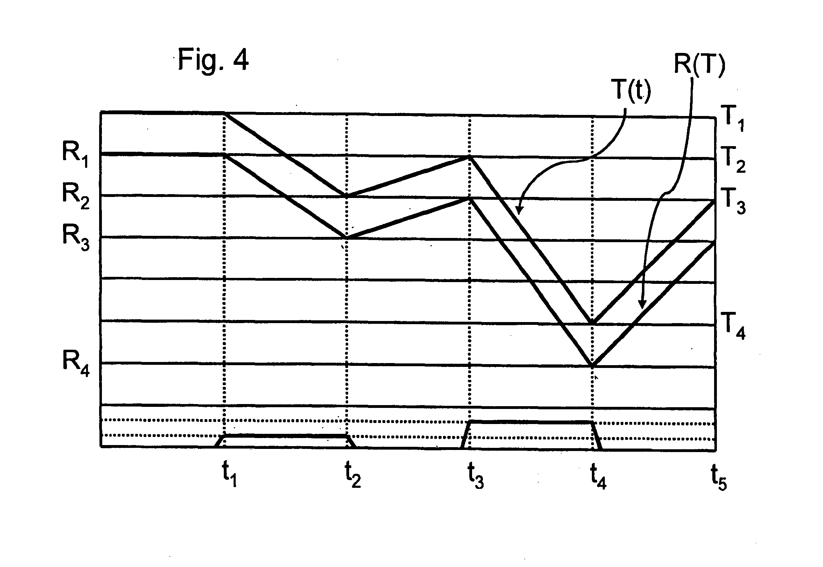

Anhand des Diagramms aus Fig. 4 wird die erfindungsgemäße Funktionsweise der Messeinrichtung 37 beschrieben.

Die X-Achse gibt den Verlauf der Zeit t in Sekunden an. Die Y-Achse gibt Impedanzwerte $R_1$, $R_2$, $R_3$, $R_4$ einer gemessenen Impedanz $R(T)$ in Ohm (untere Linie) und eine an der Neutralelektrode 10 vorliegende Temperatur $T(t)$ (obere Linie) in Grad Celsius an. Während in Y-Richtung die Temperaturwerte $T_1$, $T_2$, $T_3$, $T_4$ abnehmen, nehmen die Impedanzwerte $R_1$, $R_2$, $R_3$, $R_4$ entlang der Y-Richtung zu.

Das Diagramm zeigt beispielhaft den Verlauf einer HF-Behandlung mit einer erfindungsgemäßen Neutralelektrode 10. Unmittelbar nach dem Aufbringen der Neutralelektrode 10 stellt sich in dem Hydrogel 13 der erste Temperaturwert $T_1$ ein. Der erste Temperaturwert $T_1$ entspricht im Wesentlichen der Körperoberflächentemperatur von ca. 32 ° C. Die Messeinrichtung 37 kann den ersten Impedanzwert $R_1$ erfassen. Zum Zeitpunkt $t_1$ wird der HF-Generator 31 mit einer geringen Leistung aktiviert (Schematisch durch die Rampe im Diagramm dargestellt). Die Aktivierungsphase dauert bis zu dem Zeitpunkt $t_2$. Während der Aktivierungsphase sinkt die gemessene Impedanz $R(T)$ auf den Impedanzwert $R_3$. Da der Messeinrichtung 37 die Ausgangstemperatur $T_1$, die Ausgangsimpedanz $R_1$ und der Impedanzwert $R_3$ zum Zeitpunkt $t_2$ bekannt ist, kann sie mittels der oben genannten Formel die Temperaturänderung $\Delta T$ berechnen. Anhand dieser lässt sich der absolute Temperaturwert $T_3$ ermitteln.

Während einer Deaktivierungsphase (Zeitpunkt $t_2$ bis $t_3$) steigt die gemessene Impedanz $R(T)$. Auch hier lässt sich die Temperaturänderung $\Delta T$ anhand der Impedanzänderung $\Delta R$ ermitteln, da $R_2$ messbar ist und $R_3$, $T_3$ bekannt sind. Die Messeinrichtung 37 kann also die Temperatur $T_2$ aus der aktuellen Temperaturänderung $\Delta T$ errechnen. In einer folgenden Aktivierungsphase des HF-Generators 31 (Zeitraum $t_3$ bis $t_4$) steigt die Temperatur der Neutralelektrode $T(t)$ erneut an. Auch hier lässt sich die Temperaturänderung $\Delta T$ berechnen.

Vorab wurde ein Ausführungsbeispiel zur erfindungsgemäßen Bestimmung der Temperatur $T(t)$ und der Temperaturänderung $\Delta T$ an der Neutralelektrode 10 beschrieben.

In weiteren Ausführungsbeispielen können weitere Parameter verarbeitet werden. Beispielsweise ist es denkbar, die Temperaturänderung $\Delta T$ während eines Zeitintervalls zu berücksichtigen. So kann ein starker Temperaturabfall während einer relativ kurzen Deaktivierungsphase als Indikator verwendet werden, dass eine relativ hohe Temperatur $T(t)$ an der Neutralelektrode 10 vorliegt, da wohl ein starkes Temperaturgefälle zur Umgebung vorliegt. Zahlreiche weitere Verfahren sind denkbar, die den Effekt ausnützen, dass eine unmittelbare Korrelation zwischen der Impedanzänderung $\Delta R$ des Hydrogels 13 und dessen Temperaturänderung $\Delta T$ vorliegt.

Bezugszeichenliste

| | |
|---|---|
| 1 | Torso |
| 3 | Gewebe |
| 10 | Neutralelektrode |
| 11, 11' | Elektrodenabschnitt |
| 13 | Hydrogel |
| 20 | monopolares Instrument |
| 30 | HF-Generatorsystem |
| 31 | HF-Generator |
| 32 | Anzeigeeinrichtung |
| 34 | Bedieneinrichtung |

| 36 | Steuereinrichtung |
| 37 | Messeinrichtung |
| 38 | Sekundärstromquelle |
| 39 | Spannungsmesseinrichtung |
| $\Delta R$ | Impedanzänderung |
| $\Delta T$ | Temperaturänderung |
| $T(t)$ | Temperatur der Neutralelektrode |
| $R(T)$ | Impedanz |
| $I_{Mess}$ | Messstrom |
| $U_{Mess}$ | Messspannung |
| $R_{Gel1}$, $R_{Gel2}$ | Gelwiderstand |
| $R_{Gewebe}$ | Gewebewiderstand |
| $I_{HF}$ | HF-Strom |
| $I_{HF1}$, $I_{HF2}$ | HF-Teilstrom |
| $t_1, t_2, t_3, t_4, t_5$ | Zeitpunkt |
| $T_1, T_2, T_3, T_4$ | Temperaturwert |
| $R_1, R_2, R_3, R_4$ | Impedanzwerte |
| $T$ | Temperatur |
| $\alpha$ | Temperaturkoeffizient |

**Patentansprüche**

1. Elektrochirurgisches Gerät, umfassend:

   - einen HF-Generator (31) zum Erzeugen eines HF-Stroms ($I_{HF}$), der über ein Instrument (20) und eine Neutralelektrode (10) mit einer Kontaktmittelschicht (13) in ein biologisches Gewebe (3) einleitbar ist;
   - eine Temperaturmesseinrichtung (37) zur Bestimmung einer Temperatur und/oder einer Temperaturänderung ($\Delta T$) an der Neutralelektrode (10); wobei die Temperaturmesseinrichtung (37) zur Bestimmung der Temperatur und/oder der Temperaturänderung ($\Delta T$) eine Impedanzmesseinrichtung aufweist, die dazu ausgebildet ist, eine Impedanz ($R(T)$) der Kontaktmittelschicht (13) zu erfassen,

   wobei die Impedanzmesseinrichtung einen Messstromgenerator umfasst, der zur Bereitstellung eines Messstroms ($I_{Mess}$) an einem ersten Elektrodenabschnitt (11) und einem zweiten Elektrodenabschnitt (11') ausgebildet ist, **gekennzeichnet durch**
   eine Stromintegrationseinrichtung, die dazu ausgebildet ist, einen Wert bezüglich des HF-Stroms ($I_{HF}$), über die Zeit aufzusummieren und diesen Wert zur Bestimmung der Temperatur und/oder der Temperaturänderung ($\Delta T$) mit einer Impedanzänderung ($\Delta R$) in Beziehung zu setzen.

2. Elektrochirurgisches Gerät nach Anspruch 1, **dadurch gekennzeichnet, dass**
   der Messstromgenerator zur Bereitstellung des Messstroms ($I_{Mess}$) mit einer Wechselspannung, insbesondere mit einer Frequenz kleiner gleich 300 kHz, insbesondere kleiner gleich 150 kHz, insbesondere kleiner gleich 100 kHz ausgebildet ist.

3. Elektrochirurgisches Gerät nach einem der vorhergehenden Ansprüche,
   **dadurch gekennzeichnet, dass**
   die Elektrodenabschnitte (11, 11') elektrisch voneinander isoliert auf der Kontaktmittelschicht (13) angeordnet sind.

4. Elektrochirurgisches Gerät nach einem der vorhergehenden Ansprüche,
   **dadurch gekennzeichnet, dass**
   der HF-Generator (31) zur Bereitstellung eines HF-Stroms ($I_{HF}$) mit einer Wechselspannung mit einer Frequenz größer gleich 300 kHz, insbesondere größer gleich 1000 kHz ausgebildet ist.

5. Elektrochirurgisches Gerät nach einem der vorhergehenden Ansprüche,
   **dadurch gekennzeichnet, dass**
   die Kontaktmittelschicht (13) eine elektrische Impedanz mit einer Temperaturabhängigkeit, insbesondere mit einer relativen Impedanzänderung größer gleich 1% pro Grad Celsius, insbesondere größer gleich 2% pro Grad Celsius aufweist.

**6.** Elektrochirurgisches Gerät nach einem der vorhergehenden Ansprüche,
**dadurch gekennzeichnet, dass**
die Kontaktmittelschicht (13) Hydrogel umfasst.

**7.** Elektrochirurgisches Gerät nach einem der vorhergehenden Ansprüche,
**dadurch gekennzeichnet, dass**
die Temperaturmesseinrichtung (37) eine Impedanzintegrationseinrichtung, umfasst, die dazu ausgebildet ist Impedanzänderungen ($\Delta R$) über einen vorgegebenen Zeitraum zu integrieren, um eine Wärmebilanzabschätzung vorzunehmen.

**8.** Elektrochirurgisches Gerät nach Anspruch 7,
**dadurch gekennzeichnet, dass**
der vorgegebene Zeitraum eine Vielzahl von Aktivierungs- und Deaktivierungsphasen des HF-Generators (31) umfasst.

**9.** Elektrochirurgisches Gerät nach einem der vorhergehenden Ansprüche,
**gekennzeichnet durch**
eine Erkennungsvorrichtung zur Bestimmung von Parametern, insbesondere mindestens einer Elektrodenfläche der Neutralelektrode (10) und/oder eines Temperaturkoeffizienten ($\alpha$).

**10.** Elektrochirurgisches Gerät nach Anspruch 9,
**dadurch gekennzeichnet, dass**
die Erkennungsvorrichtung eine Datenbank mit einer Vielzahl von Parametern und einer Vielzahl von Neutralelektrodentypen umfasst, wobei die Erkennungsvorrichtung dazu ausgebildet ist, ein Anschließen eines bestimmten Neutralelektrodentypen zu erfassen und die Parameter demgemäß aus der Datenbank auszulesen.

**11.** Elektrochirurgisches Gerät nach einem der vorhergehenden Ansprüche,
**gekennzeichnet durch**
eine Unterbrechungseinrichtung, die dazu ausgebildet ist, beim Überschreiten einer vorgegebenen Impedanzänderung den HF-Strom ($I_{HF}$) zu unterbrechen oder zu limitieren.

**12.** Elektrochirurgisches Gerät nach einem der vorhergehenden Ansprüche,
**dadurch gekennzeichnet, dass**
die Kontaktmittelschicht (13) eine derartige Materialeigenschaft aufweist, dass deren Impedanz mit zunehmender Temperatur abnimmt.

**13.** Elektrochirurgisches Gerät nach einem der vorhergehenden Ansprüche,
**dadurch gekennzeichnet, dass**
die Temperaturmesseinrichtung (37) zur Bestimmung der Temperatur und/oder der Temperaturveränderung den Effektivwert des HF-Stroms ($I_{HF}$) berücksichtigt.

**14.** Elektrochirurgisches Gerät nach einem der vorhergehenden Ansprüche,
**dadurch gekennzeichnet, dass**
die Stromintegrationseinrichtung dazu ausgebildet ist, den Effektivwert des HF-Stroms ($I_{HF}$) über die Zeit aufzusummieren.

**Claims**

**1.** Electrosurgical device, comprising:

- an RF generator (31) for generating an RF current ($I_{HF}$), which can be introduced into biological tissue (3) via an instrument (20) and a neutral electrode (10) with a contact agent layer (13) ;
- a temperature measuring apparatus (37) for determining a temperature and/or a change in temperature ($\Delta T$) at the neutral electrode (10); the temperature measuring apparatus (37) comprising an impedance measuring apparatus for determining the temperature and/or the change in temperature ($\Delta T$), said impedance measuring apparatus being embodied to register an impedance ($R(T)$) of the contact agent layer (13),

the impedance measuring apparatus comprising a measurement current generator, which is embodied to provide a measurement current ($I_{Mess}$) at a first electrode section (11) and a second electrode section (11'),
**characterized by**
a current integration apparatus, which is embodied to sum a value in respect of the RF current ($I_{HF}$) over time and to relate this value to a change in impedance ($\Delta R$) in order to determine the temperature and/or the change in temperature ($\Delta T$).

2. Electrosurgical device according to Claim 1,
**characterized in that**
the measurement current generator is embodied to provide the measurement current ($I_{Mess}$) with an AC voltage, in particular with a frequency of less than or equal to 300 kHz, in particular of less than or equal to 150 kHz, in particular of less than or equal to 100 kHz.

3. Electrosurgical device according to one of the preceding claims,
**characterized in that**
the electrode sections (11, 11') are arranged on the contact agent layer (13) in a manner electrically insulated from one another.

4. Electrosurgical device according to one of the preceding claims,
**characterized in that**
the RF generator (31) is embodied to provide an RF current ($I_{HF}$) with an AC voltage with a frequency of greater than or equal to 300 kHz, in particular of greater than or equal to 1000 kHz.

5. Electrosurgical device according to one of the preceding claims,
**characterized in that**
the contact agent layer (13) has an electrical impedance with a temperature dependence, in particular with a relative change in impedance of greater than or equal to 1% per degree centigrade, in particular of greater than or equal to 2% per degree centigrade.

6. Electrosurgical device according to one of the preceding claims,
**characterized in that**
the contact agent layer (13) comprises a hydrogel.

7. Electrosurgical device according to one of the preceding claims,
**characterized in that**
the temperature measuring apparatus (37) comprises an impedance integration apparatus, which is embodied to integrate changes in impedance ($\Delta R$) over a predetermined period of time in order to perform a heat balance estimate.

8. Electrosurgical device according to Claim 7,
**characterized in that**
the predetermined period of time comprises a multiplicity of activation and deactivation phases of the RF generator (31).

9. Electrosurgical device according to one of the preceding claims,
**characterized by**
identification equipment for determining parameters, in particular of at least one electrode area of the neutral electrode (10) and/or of a temperature coefficient ($\alpha$).

10. Electrosurgical device according to Claim 9,
**characterized in that**
the identification equipment comprises a database with a multiplicity of parameters and a multiplicity of neutral electrode types, the identification equipment being embodied to register a specific neutral electrode type being connected and accordingly read out the parameters from the database.

11. Electrosurgical device according to one of the preceding claims,
**characterized by**
a cut-off apparatus, which is embodied to cut off or limit the RF current ($I_{HF}$) when a predetermined change in impedance is exceeded.

**12.** Electrosurgical device according to one of the preceding claims,
**characterized in that**
the contact agent layer (13) has such a material property that the impedance thereof decreases with increasing temperature.

**13.** Electrosurgical device according to one of the preceding claims,
**characterized in that**
the temperature measuring apparatus (37) for determining the temperature and/or the change in temperature takes the effective value of the RF current ($I_{HF}$) into account.

**14.** Electrosurgical device according to one of the preceding claims,
**characterized in that**
the current integration apparatus is embodied to sum the effective value of the RF current ($I_{HF}$) over time.


**Revendications**

**1.** Appareil électrochirurgical comprenant :

- un générateur HF (31) qui produit un courant HF ($I_{HF}$) qui peut être injecté dans un tissu biologique (3) par l'intermédiaire d'un instrument (20) et d'une électrode neutre (10) dotée d'une couche (13) de moyen de contact,
- un dispositif (37) de mesure de température qui détermine la température et/ou la modification ($\Delta T$) de la température au niveau de l'électrode neutre (10),

le dispositif (37) de mesure de température présentant pour déterminer la température et/ou la modification ($\Delta T$) de la température un dispositif de mesure d'impédance configuré pour saisir l'impédance ($R(T)$) de la couche (13) de moyen de contact,
le dispositif de mesure d'impédance comportant un générateur de courant de mesure qui est configuré pour délivrer un courant de mesure ($I_{Mess}$) sur une première partie (11) de l'électrode et une deuxième partie (11') de l'électrode,
**caractérisé par**
un dispositif d'intégration de courant configuré pour additionner au cours du temps une valeur qui concerne le courant HF ($I_{HF}$) et pour mettre cette valeur en relation avec une modification d'impédance ($\Delta R$) pour déterminer la température et/ou la modification ($\Delta T$) de la température.

**2.** Appareil électrochirurgical selon la revendication 1, **caractérisé en ce que** le générateur de courant de mesure est configuré pour délivrer le courant de mesure ($I_{Mess}$) sous une tension alternative, en particulier à une fréquence inférieure ou égale à 300 kHz, en particulier inférieure ou égale à 150 kHz et notamment inférieure ou égale à 100 kHz.

**3.** Appareil électrochirurgical selon l'une des revendications précédentes, **caractérisé en ce que** les parties (11, 11') de l'électrode sont isolées électriquement l'une de l'autre et sont disposées sur la couche (13) de moyen de contact.

**4.** Appareil électrochirurgical selon l'une des revendications précédentes, **caractérisé en ce que** le générateur HF (31) est configuré pour délivrer un courant HF ($I_{HF}$) sous une tension alternative d'une fréquence supérieure ou égale à 300 kHz et notamment supérieure ou égale à 1 000 kHz.

**5.** Appareil électrochirurgical selon l'une des revendications précédentes, **caractérisé en ce que** la couche (13) de moyen de contact présente une impédance électrique qui dépend de la température et notamment dont la modification relative de l'impédance est supérieure ou égale à 1 % par degré Celsius et en particulier supérieure ou égale à 2 % par degré Celsius.

**6.** Appareil électrochirurgical selon l'une des revendications précédentes, **caractérisé en ce que** la couche (13) de moyen de contact comporte un hydrogel.

**7.** Appareil électrochirurgical selon l'une des revendications précédentes, **caractérisé en ce que** le dispositif (37) de mesure de température comporte un dispositif d'intégration d'impédance configuré pour intégrer les modifications d'impédance ($\Delta R$) pendant une durée prédéterminée pour réaliser une estimation du bilan thermique.

**8.** Appareil électrochirurgical selon la revendication 7, **caractérisé en ce que** la durée prédéterminée comporte plusieurs phases d'activation et de désactivation du générateur HF (31).

9. Appareil électrochirurgical selon l'une des revendications précédentes, **caractérisé par** un dispositif de détection qui détermine des paramètres et en particulier au moins la surface de l'électrode neutre (10) et/ou un coefficient de température ($\alpha$).

10. Appareil électrochirurgical selon la revendication 9, **caractérisé en ce que** le dispositif de détection comporte une base de données qui contient plusieurs paramètres et plusieurs types d'électrodes neutres, le dispositif de détection étant configuré pour détecter le raccordement d'un type défini d'électrode neutre et pour lire les paramètres dans la base de données en fonction de cette détection.

11. Appareil électrochirurgical selon l'une des revendications précédentes, **caractérisé par** un dispositif d'interruption configuré pour interrompre ou limiter le courant HF ($I_{HF}$) lorsqu'une modification prédéterminée de l'impédance est dépassée.

12. Appareil électrochirurgical selon l'une des revendications précédentes, **caractérisé en ce que** la couche (13) de moyen de contact présente un matériau dont les propriétés sont telles que son impédance diminue lorsque la température augmente.

13. Appareil électrochirurgical selon l'une des revendications précédentes, **caractérisé en ce que** le dispositif (37) de mesure de température tient compte de la valeur efficace du courant HF ($I_{HF}$) pour déterminer la température et/ou la modification de température.

14. Appareil électrochirurgical selon l'une des revendications précédentes, **caractérisé en ce que** le dispositif d'intégration de courant est configuré pour additionner au cours du temps la valeur efficace du courant HF ($I_{HF}$).

Fig. 1

30    20    1    10

Fig. 2

30

32

31    36    34

37
38

Fig. 3

39    38

10
11    11'
$I_{HF1}$    $I_{HF2}$
$I_{HF}$
$R_{Gel2}$    $R_{Gel1}$
13
$R_{Mess}$    $R_{Gewebe}$
3

Fig. 4

EP 2 367 493 B1

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 102004025613 B4
- US 20060079872 A1
- EP 1707151 A2